# EUROPEAN PATENT SPECIFICATION

(11) **EP 2 622 065 B1**
(45) Date of publication and mention of the grant of the patent: **07.09.2016**
(21) Application number: 11771334.7
(22) Date of filing: 30.09.2011
(51) Int. Cl.: C12N 15/85, C12N 5/074

(54) **MANIPULATION OF STEM CELL FUNCTION BY P53 ISOFORMS**
MANIPULATION DER STAMMZELLENFUNKTION MIT P53-ISOFORMEN
MANIPULATION DE LA FONCTION DE CELLULES SOUCHES PAR DES ISOFORMES P53

(30) Priority: 01.10.2010 US 389134 P
(43) Date of publication of application: 07.08.2013
(73) Proprietor: The Government of the United States of America as represented by the Secretary of the Department of Health and Human Services, Rockville, MD 20852-3804 (US); Fundación Centro Nacional de Investigaciones Oncológicas Carlos III, 28029 Madrid (ES)
(72) Inventor: HARRIS, Curtis C., Maryland 20892 (US); HORIKAWA, Izumi, Maryland 20892 (US); FUJITA, Kaori, Maryland 20892 (US); MONDAL, Abdul M., Maryland 20892 (US); PARK, Kye-Yoon, Maryland 20892 (US); PINE, Sharon R., Maryland 20892 (US); SERRANO, Manuel, Maryland 20892 (US)
(74) Representative: Campbell, Patrick John Henry
(86) International application number: PCT/US2011/054304
(87) International publication number: WO 2012/044979

(56) References cited:
- WO-A1-2009/029054
- WO-A1-2009/157593
- WO-A2-2009/064590
- KAORI FUJITA ET AL: "p53 isoforms [Delta]133p53 and p53[beta] are endogenous regulators of replicative cellular senescence", NATURE CELL BIOLOGY, vol. 11, no. 9, 1 September 2009 (2009-09-01), pages 1135-1142, XP55016287, ISSN: 1465-7392, DOI: 10.1038/ncb1928
- TERUHISA KAWAMURA ET AL: "Linking the p53 tumour suppressor pathway to somatic cell reprogramming", NATURE: INTERNATIONAL WEEKLY JOURNAL OF SCIENCE, NATURE PUBLISHING GROUP, UNITED KINGDOM, vol. 460, no. 7259, 1 August 2009 (2009-08-01), pages 1140-1145, XP002639994, ISSN: 0028-0836, DOI: 10.1038/NATURE08311
- HYENJONG HONG ET AL: "Suppression of induced pluripotent stem cell generation by the p53-p21 pathway", NATURE: INTERNATIONAL WEEKLY JOURNAL OF SCIENCE, NATURE PUBLISHING GROUP, UNITED KINGDOM, vol. 460, 1 August 2009 (2009-08-01), pages 1132-1135, XP008129610, ISSN: 0028-0836, DOI: 10.1038/NATURE08235
- TAKENAKA C ET AL: "Effective generation of iPS cells from CD34<+> cord blood cells by inhibition of p53", EXPERIMENTAL HEMATOLOGY, ELSEVIER INC, US, vol. 38, no. 2, 1 February 2010 (2010-02-01), pages 154-162.E2, XP026855304, ISSN: 0301-472X, DOI: 10.1016/J.EXPHEM.2009.11.003 [retrieved on 2009-11-14]
- V MARCEL ET AL: "p53 regulates the transcription of its [Delta]133p53 isoform through specific response elements contained within the TP53 P2 internal promoter", ONCOGENE, vol. 29, no. 18, 6 May 2010 (2010-05-06), pages 2691-2700, XP55016333, ISSN: 0950-9232, DOI: 10.1038/onc.2010.26
- SONG WEI ET AL: "Expression of p53 isoforms in renal cell carcinoma", CHINESE MEDICAL JOURNAL (ENGLISH EDITION), vol. 122, no. 8, April 2009 (2009-04), pages 921-926, XP002671567, ISSN: 0366-6999
- BOURDON JEAN-CHRISTOPHE ET AL: "p53 isoforms can regulate p53 transcriptional activity", GENES & DEVELOPMENT, vol. 19, no. 18, September 2005 (2005-09), pages 2122-2137, XP002671568, ISSN: 0890-9369
- SLATTER TANIA L ET AL: "Hyperproliferation, cancer, and inflammation in mice expressing a Delta 133p53-like isoform", BLOOD, vol. 117, no. 19, May 2011 (2011-05), pages 5166-5177, XP002671569,
- MARCEL V ET AL: "DELTA160p53 is a novel N-terminal p53 isoform encoded by DELTA133p53 transcript", FEBS LETTERS, ELSEVIER, AMSTERDAM, NL, vol. 584, no. 21, 5 November 2010 (2010-11-05), pages 4463-4468, XP027446892, ISSN: 0014-5793, DOI: 10.1016/J.FEBSLET.2010.10.005 [retrieved on 2010-10-13]

## Description

The activity of p53 regulates the self-renewal and pluripotency of normal and cancer stem cells, as well as the re-programming efficiency of induced pluripotent stem (iPS) cells *(see, e.g.,* Mario et al, Nature 460:1149, 2009; Hong et al, Nature 460:1132, 2009; Cicalese et al., Cell 138:1083, 2009). Natural human p53 isoforms Δ133p53 and p53β are the physiological inhibitor and enhancer, respectively, of p53 activity (*see, e.g.,* Fujita et al, Nat. Cell Biol. 11:1135, 2009).

Human iPS cells are derived from somatic cells and are typically produced by expression of Oct-3/4, Sox- 2, c-Myc, and Klf-4 or by Oct-3/4, Sox-2, Nanog, and Lin28. These cells are morphologically similar to human embryonic stem cells, express typical human ESC-specific cell surface antigens and genes, differentiate into multiple lineages *in vitro,* and form teratomas containing differentiated derivatives of all three primary germ layers when injected into immunocompromised mice. However, the efficiency of reprogramming adult somatic cells such as fibroblasts to obtain iPS cells is fairly low. Inhibition of p53 activity improves the efficiency of iPS cell reprogramming; however, total inhibition of wild-type p53 activity has a risk of inducing genome instability and excess over-expression of wild-type p53 can induce unwanted cell death. Thus, there is a need for improved methods of regulating iPS cell re-programming that targets p53 pathways without the risks associated with genome instability. This invention addresses that need.

Fujita et al (2009) Nature Cell Biology, 11, 1135-1142 states that it discloses that the human p53 isoforms Δ133p53 and p53β function in an endogenous regulatory mechanism for p53-mediated replicative senescence. Kawamura et al (2009) Nature, 460, 1140-1145 states that it discloses an investigation into the mechanisms limiting reprogramming efficiency in somatic cells. Hong et al (2009) Nature, 460, 1132-1135 states that it discloses that up to 10% of transduced mouse embryonic fibroblasts lacking p53 become iPS cells even without the Myc retrovirus. WO 2009/157593 states that it discloses a method of improving the efficiency of establishment of induced pluripotent stem cells comprising inhibiting p53 function in the step of somatic nuclear reprogramming. Takenaka et al (2010) Experimental Hematology, 38, 154-162 states that it discloses that iPS cells can be generated easily from CD34⁺ cord blood cells through the addition of p53 inhibition to standard reprogramming conditions.

### BRIEF SUMMARY OF THE INVENTION

The invention is based, in part, on the discovery that human embryonic stem cells (hESC) express Δ133p53 protein much more abundantly than normal human fibroblasts or cancer cell lines; and further, on the discovery that overexpression of Δ133p53 enhances the efficiency of iPS cell generation. Thus, in one aspect, the claimed invention provides a method of increasing the number of induced pluripotent stem cells obtained from somatic cells that express at least one reprogramming factor, the method comprising increasing the Δ133p53 levels in the somatic cells, thereby increasing the number of iPS cells obtained from the somatic cells. In some embodiments, the level of expression of Δ133p53 is increased by expressing a recombinant nucleic acid sequence that encodes Δ133p53 in the somatic cells. In some embodiments, the recombinant nucleic acid sequence that encodes Δ133p53 is operably linked to an inducible promoter. In some embodiments, the nucleic acid that encodes Δ133p53 is an RNA molecule. In some embodiments, the nucleic acid introduced into the cell is contained in a vector, *e.g.,* a plasmid vector, an adenoviral vector, a retroviral vector, or any other type of viral vector. In some embodiments, the recombinant nucleic acid is present in an expression cassette that comprises lox recombination sites.

In some embodiments, the cells that are being re-programmed to obtain iPS cells for use in the invention are fibroblasts; or other partially- or fully-differentiated cells *(e.g.,* blood cells, dermal cells, epithelial cells, keratinocytes, and the like.

In some embodiments, the level of expression of Δ133p53 is increased by introducing exogenous Δ133p53 into the cell.

In the claimed invention, the cells that are being reprogrammed that are employed in the methods of the invention express at least one reprogramming factor, *e.g.,* at least one reprogramming factor selected from: OCT4, KLF4, SOX2, and c-myc. In some embodiments, the somatic cells express OCT4, KLF4, and SOX2. In some embodiments, the somatic cells express OCT4, KLF4, SOX2, and c-myc.

In another aspect, the disclosure provides an isolated iPS cell comprising a heterologous nucleic acid encoding Δ133p53, *e.g.* an expression vector encoding a recombinant Δ133p53, wherein the iPS cell expresses at least one re-programming growth factor, *e.g.,* OCT4, KLF4, SOX2, or c-myc. In some instances, the heterologous nucleic acid that encodes Δ133p53 is operably linked to an inducible promoter. In some instances, the nucleic acid is an RNA. In some instances, the heterologous nucleic acid is a Δ133p53 expression vector. The expression vector can be any kind of vector, *e.g.,* a plasmid vector or a viral vector, such as an adenoviral vector or a retroviral vector, *e.g.,* a lentiviral vector. In some instances, the iPs cell expresses OCT4, KLF4, and SOX2. In some instances, the iPS cell expresses OCT4, KLF4, SOX2, and c-myc. In some instances, the expression vector comprises a lox recombination site. In one aspect, the claimed invention provides an isolated iPS cell comprising an expression vector encoding a recombinant Δ133p53, wherein the iPS cell expresses at least one re-programming growth factor.

In another aspect, the invention provides a method of differentiating an iPS cell of the invention that expresses Δ133p53, the method comprising suppressing the expression of Δ133p53. In some embodiments in which the promoter that drives expression of Δ133p53 is an inducible promoter, the cell is no longer exposed to the inducing agent. In some embodiments in which the expression vector comprises a lox recombination site, a cre recombinase is expressed in the cell to inactivate Δ133p53 expression. In some embodiments, the iPS cell is differentiated into a neuron, cardiomyocyte, hepatocyte, or lung respiratory epithelial cell.

In a further aspect, the disclosure provides a method of inhibiting growth of a cancer stem cell, the method comprising inhibiting expression of Δ133p53 in a cancer stem cell. Expression may be inhibited, e.g., using an inhibitory RNA.

In another aspect, the disclosure provides a method of inhibiting growth of a cancer stem cell, the method comprising increasing expression of p53β. In some instances, the method comprises introducing a p53β expression construct into the cancer stem cell.

In a further aspect, the disclosure provides a method of increasing the number of inducible pluripotent (iPS) stem cells obtained from somatic cells that express at least one reprogramming factor, the method comprising decreasing the level of expression of p53β in the cells, thereby increasing the number of iPS obtained. In some instances, the level of expression of p53β is decreased by expressing a recombinant nucleic acid sequence that inhibits p53β, *e.g.,* an inhibitory RNA, in the cells. In some instances, the cells that undergo reprogramming are fibroblasts; or other partially- or fully-differentiated cell *(e.g.,* blood cells, dermal cells, epithelial cells, keratinocytes, and the like). In some instances, the cells that undergo reprogramming express at least one reprogramming factor selected from: OCT4, KLF4, SOX2, and c-myc. In some instances, the somatic cells express OCT4, KLF4, and SOX2. In some instances, the somatic cells express OCT4, KLF4, SOX2, and c-myc.

### BRIEF DESCRIPTION OF THE DRAWINGS

Figure 1 provides data showing that Δ133p53 is upregulated in embryonic stem cells.
Figure 2 provides further data showing that either downregulation of endogenous full-length p53 (clone 3, cl.3) or upregulation of endogenous Δ133p53 (clone 2, cl. 2) may be associated with iPS cell reprogramming. The iPS cell clones analyzed in this experiment were induced by 4 factors (Oct4, Klf4, Sox2, and c-Myc).
Figure 3A and 3B provide data showing that Δ133p53 increased the frequency of induced pluripotent cells that are obtained from BJ fibroblasts induced by three iPS cells factors (OKS: Oct4, Klf4, and Sox2) or four iPS cell factors (OKSM: Oct4, Klf4, Sox2, and c-Myc).
Figure 4 shows the cell morphology of iPS cells that were derived from BJ fibroblasts transduced with the retroviral vectors of four iPS factors (OKSM: Oct4, Klf4, Sox2, and c-Myc) and Δ133p53 overexpression.
Figure 5 provides data showing constitutive overexpression of Δ133p53 using a lentiviral vector system.
Figure 6 provides data showing establishment of an inducible lentiviral vector system for Δ133p53 expression.
Figure 7 provides data showing the results of knockdown experiments obtained using five independent knockdown vectors targeting different sequences.

### DETAILED DESCRIPTION OF THE INVENTION

### Definitions

The term "pluripotent" or "pluripotency" as used herein refers to cells with the ability to give rise to progeny that can undergo differentiation, under the appropriate conditions, into cell types that collectively demonstrate characteristics associated with cell lineages from all of the three germinal layers (endoderm, mesoderm, and ectoderm). Pluripotent stem cells can contribute to many or all tissues of a prenatal, postnatal or adult animal. A standard art-accepted test, such as the ability to form a teratoma in 8-12 week old SCID mice, can be used to establish the pluripotency of a cell population, however identification of various pluripotent stem cell characteristics can also be used to detect pluripotent cells. Cell pluripotency is a continuum, ranging from the completely pluripotent cell that can form every cell of the embryo proper, e.g., embyronic stem cells and iPSCs, to the incompletely or partially pluripotent cell that can form cells of all three germ layers but that may not exhibit all the characteristics of completely pluripotent cells, such as, for example, germline transmission or the ability to generate a whole organism. In particular embodiments of the invention, the pluripotency of a cell is increased from an incompletely or partially pluripotent cell to a more pluripotent cell or, in certain embodiments, a completely pluripotent cell. Pluripotency can be assessed, for example, by teratoma formation, germ-line transmission, and tetraploid embryo complementation. In some instances, expression of pluripotency genes or pluripotency markers as discussed elsewhere herein, can be used to assess the pluripotency of a cell.

"Pluripotent stem cell characteristics" refer to characteristics of a cell that distinguish pluripotent stem cells from other cells. The ability to give rise to progeny that can undergo differentiation, under the appropriate conditions, into cell types that collectively demonstrate characteristics associated with cell lineages from all of the three germinal layers (endoderm, mesoderm, and ectoderm) is a pluripotent stem cell characteristic. Expression or non-expression of certain combinations of molecular markers are also pluripotent stem cell characteristics. For example, human pluripotent stem cells express at least some, and optionally all, of the markers from the following non -limiting list: SSEA-3, SSEA-4, TRA-1-60, TRA-1-81, TRA-2-49/6E, ALP, Sox2, E-cadherin, UTF-1, Oct4, Rex1, and Nanog. Cell morphologies associated with pluripotent stem cells are also pluripotent stem cell characteristics.

A "somatic cell" as used herein refers to differentiated, or partially differentiated cells relative to embryonic stem cells. Thus, the term includes, *e.g.,* cells such as fibroblasts that are derived from embryonic stem cells, but are differentiated.

The term "embryonic stem cell" is used to refer to the pluripotent stem cells of the inner cell mass of the embryonic blastocyst (see US Patent Nos. 5843780, 6200806). The distinguishing characteristics of an embryonic stem cell define an embryonic stem cell phenotype. Accordingly, a cell has the phenotype of an embryonic stem cell if it possesses one or more of the unique characteristics of an embryonic stem cell such that that cell can be distinguished from other cells. Illustrative distinguishing embryonic stem cell characteristics include, without limitation, gene expression profile, proliferative capacity, differentiation capacity, normal karyotype, responsiveness to particular culture conditions, and the like.

A "cancer stem cell" as used herein refers to self-renewing and pluripotent cancer cells (see, *e.g.,* U.S. Patent No. 6,984,522). Such cells can be obtained from any tumor source including primary or any metastatic tumor site, lymph nodes, ascites fluids, or blood. Cancer stem cells are identified by virtue of their functional characteristics that include, without limitation, the ability to repopulate new tumors in serial transplants, and ability to give rise to the functional and phenotypic cellular heterogeneity of the original tumor.

In the context of this disclosure, "inhibiting proliferation of a cancer stem cell" refers to reducing the growth of the cancer stem cell by any mechanism including by inducing apoptosis, increasing senescence and/or decreasing the rate of cell growth, i.e., the cell cycle transit time.

The term "reprogramming" as used herein refers to the process that alters or reverses the differentiation state of a somatic cell. The cell can either be partially or terminally differentiated prior to the reprogramming. Reprogramming encompasses complete reversion of the differentiation state of a somatic cell to a pluripotent cell. Such complete reversal of differentiation can produce an induced pluripotent (iPS) cell. Reprogramming as used herein also encompasses partial reversion of a cells differentiation state, for example to a multipotent state or to a somatic cell that is neither pluripotent or multipotent, but is a cell that has lost one or more specific characteristics of the differentiated cell from which it arises, e.g. direct reprogramming of a differentiated cell to a different somatic cell type. Reprogramming generally involves alteration, e.g., reversal, of at least some of the heritable patterns of nucleic acid modification (e.g., methylation), chromatin condensation, epigenetic changes, genomic imprinting, etc., that occur during cellular differentiation as a zygote develops into an adult.

The term "p53" refers generally to a protein of a molecular weight of about 55kDa on SDS PAGE that functions as a tumor suppressor. The protein and nucleic sequences of the p53 protein from a variety of organisms from humans to *Drosophila* are known and are available in public databases, such as in accession numbers, NM_000546, NP_000537, NM_011640, and NP_035770, for the human and mouse sequences. Mammalian p53 sequences are highly conserved between species. Mouse and human p53 proteins are 85% identical. In humans, p53 is encoded by the TP53 gene located on the short arm of chromosome 17 (17p13.1). A p53 protein in the context of this invention includes allelic variants and other functional variants and orthologs. In some embodiments, variants have at least 85%, at least 90%, or at least 95%, or greater, amino acid sequence identity across their whole sequence compared to a naturally occurring p53 family member such as that listed under accession number NP_000537 (human p53). Generally, the same species of protein will be used with the species of cells being manipulated.

The term "Δ133p53" refers generally to the isoform of p53 that arises from initiation of transcription of the p53 gene from an alternative promoter in the intron 4, which results in an N-terminally truncated p53 protein translated from codon 133. A Δ133p53 isoform for use in this invention comprises the following p53 protein domains: the majority of the DNA binding domain, the NLS, and the C-terminal oligomerization domain *(see* Bourdon, Brit. J. Cancer, 97: 277-282 (2007)).

The term "p53β" refers generally to the isoform of p53 that arises from alternative splicing of intron 9 to provide a p53 isoform comprising the following p53 protein domains: TAD1, TAD2, prD, the DNA binding domain, the NLS, and the C-terminal sequence DQTSFQKENC (*see* Bourdon, Brit. J. Cancer, 97: 277-282 (2007)).

As used herein, the term "increasing the number of induced pluripotent stems cells" is used interchangeably with "enhancing the efficiency of obtaining pluripotent stem cells". In the context of the invention, the "increase" in the number of iPS cells obtained from a population of cells, typically adult somatic cells, that are re-programmed to produce iPS when Δ133p53 is expressed along with other re-programming factors is in comparison to a control population of the same cells in which Δ133p53 is not expressed. Thus, in certain embodiments, the methods of the instant invention provide for enhancement of efficiency of iPS cell production by at least 20%, or o at least 30%, at least 40%, at least 50%, or at least 100%, or greater, *e.g.,* 3-fold, 4-fold, 5-fold, or 10-fold or greater, in comparison to somatic cell populations that are re-programmed that do not have enhanced expression of Δ133p53.

As used herein, the term "expression" or "increasing expression" of Δ133p53 in the context of this invention typically refers to introducing a Δ133p53 nucleic acid into a somatic cell that is, or will be, manipulated to undergo re-programming. In some embodiments, the level of expression of Δ133p53 may be increased using another agent that upregulates expression. An "increase" in Δ133p53 expression is generally determined relative to somatic cells that are reprogrammed to which an agent to increase levels of Δ133p53 has not been added.

A polynucleotide sequence is "heterologous to" a second polynucleotide sequence if it originates from a foreign species, or, if from the same species, is modified by human action from its original form. For example, a promoter operably linked to a heterologous coding sequence refers to a coding sequence from a species different from that from which the promoter was derived, or, if from the same species, a coding sequence which is different from any naturally occurring allelic variants.

The term "exogenous" refers to a substance present in a cell or organism other than its native source. For example, the terms "exogenous nucleic acid" or "exogenous protein" refer to a nucleic acid or protein that has been introduced by a process involving the hand of man into a biological system such as a cell or organism in which it is not normally found or in which it is found in lower amounts. A substance will be considered exogenous if it is introduced into a cell or an ancestor of the cell that inherits the substance. In contrast, the term "endogenous" refers to a substance that is native to the biological system.

The term "identity" as used herein refers to the extent to which the sequence of two or more nucleic acids or polypeptides is the same. The percent identity between a sequence of interest and a second sequence over a window of evaluation, e.g., over the length of the sequence of interest, may be computed by aligning the sequences, determining the number of residues (nucleotides or amino acids) within the window of evaluation that are opposite an identical residue allowing the introduction of gaps to maximize identity, dividing by the total number of residues of the sequence of interest or the second sequence (whichever is greater) that fall within the window, and multiplying by 100. When computing the number of identical residues needed to achieve a particular percent identity, fractions are to be rounded to the nearest whole number. Percent identity can be calculated with the use of a variety of computer programs known in the art. For example, computer programs such as BLAST2, BLASTN, BLASTP, Gapped BLAST, etc., generate alignments and provide percent identity between sequences of interest. The algorithm of Karlin and Altschul (Karlin and Altschul, Proc. Natl. Acad. ScL USA 87:22264-2268, 1990) modified as in Karlin and Altschul, Proc. Natl. Acad. ScL USA 90:5873-5877, 1993 is incorporated into the NBLAST and XBLAST programs of Altschul et al. (Altschul, et al., J. MoI. Biol. 215:403-410, 1990). To obtain gapped alignments for comparison purposes, Gapped BLAST is utilized as described in Altschul et al. (Altschul, et al. Nucleic Acids Res. 25: 3389-3402, 1997). When utilizing BLAST and Gapped BLAST programs, the default parameters of the respective programs may be used. A PAM250 or BLOSUM62 matrix may be used. Software for performing BLAST analyses is publicly available through the National Center for Biotechnology Information (NCBI). See the Web site having URL world-wide web address of: "ncbi.nlm.nih.gov" for these programs. In a specific embodiment, percent identity is calculated using BLAST2 with default parameters as provided by the NCBI.

The term "isolated" or "partially purified" as used herein refers, in the case of a nucleic acid or polypeptide, to a nucleic acid or polypeptide separated from at least one other component (e.g., nucleic acid or polypeptide) that is present with the nucleic acid or polypeptide as found in its natural source and/or that would be present with the nucleic acid or polypeptide when expressed by a cell, or secreted in the case of secreted polypeptides. A chemically synthesized nucleic acid or polypeptide or one synthesized using in vitro transcription/translation is considered "isolated".

The term "isolated cell" as used herein refers to a cell that has been removed from an organism in which it was originally found or a descendant of such a cell. An "isolated" cell may be cultured *in vitro* in the presence of other cells. Optionally, the cell is later introduced into a second organism or re-introduced into the organism from which it (or the cell from which it is descended) was isolated.

The term "vector" refers to a carrier DNA molecule into which a DNA sequence can be inserted for introduction into a host cell. In some embodiments, vectors of use in the invention are those capable of autonomous replication and/or expression of nucleic acids to which they are linked. Vectors capable of directing the expression of genes to which they are operatively linked are referred to herein as "expression vectors". Thus, an "expression vector" is a specialized vector that contains the necessary regulatory regions needed for expression of a gene of interest in a host cell. In some embodiments the gene of interest is operably linked to another sequence in the vector, *e.g.,* a promoter. Vectors include non-viral vectors such as plasmids and viral vectors.

The term "operably linked" refers to a functional linkage between a first nucleic acid sequence and a second nucleic acid sequence, such that the first and second nucleic acid sequences are transcribed into a single nucleic acid sequence. Operably linked nucleic acid sequences need not be physically adjacent to each other. The term "operably linked" also refers to a functional linkage between a nucleic acid expression control sequence (such as a promoter, or array of transcription factor binding sites) and a transcribable nucleic acid sequence, wherein the expression control sequence directs transcription of the nucleic acid corresponding to the transcribable sequence.

The term "viral vectors" refers to the use of viruses, or virus-associated vectors as carriers of a nucleic acid construct into a cell. Constructs may be integrated and packaged into non-replicating, defective viral genomes like Adenovirus, Adeno-associated virus (AAV), or Herpes simplex virus (HSV) or others, including retroviral and lentiviral vectors, for infection or transduction into cells. The vector may or may not be incorporated into the cell's genome. The constructs may include viral sequences for transfection, if desired. Alternatively, the construct may be incorporated into vectors capable of episomal replication, e.g EPV and EBV vectors.

In the context of this invention, "transfection" is used to refer to any method of introducing nucleic acid molecules into a cell including both viral and non-viral techniques. The term thus includes techniques such as transduction with a virus.

The terms "regulatory sequence" and "promoter" are used interchangeably herein, and refer to nucleic acid sequences, such as initiation signals, enhancers, and promoters, which induce or control transcription of protein coding sequences with which they are operatively linked. In some examples, transcription of a recombinant gene is under the control of a promoter sequence (or other transcriptional regulatory sequence) which controls the expression of the recombinant gene in a cell- type in which expression is intended. It will also be understood that the recombinant gene can be under the control of transcriptional regulatory sequences which are the same or which are different from those sequences which control transcription of the naturally-occurring form of a protein. In some instances the promoter sequence is recognized by the synthetic machinery of the cell, or introduced synthetic machinery, required for initiating transcription of a specific gene.

As used herein, the term "transcription factor" refers to a protein that binds to specific parts of DNA using DNA binding domains and is part of the system that controls the transfer (or transcription) of genetic information from DNA to RNA.

As used herein, "proliferating" and "proliferation" refer to an increase in the number of cells in a population (growth) by means of cell division. Cell proliferation is generally understood to result from the coordinated activation of multiple signal transduction pathways in response to the environment, including growth factors and other mitogens. Cell proliferation may also be promoted by release from the actions of intra- or extracellular signals and mechanisms that block or negatively affect cell proliferation.

The terms "nucleic acid" and "polynucleotide" are used interchangeably herein to refer to deoxyribonucleotides or ribonucleotides and polymers thereof in either single- or doublestranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)).

The term "siRNA" refers to a nucleic acid that forms a double stranded RNA, which double stranded RNA has the ability to reduce or inhibit expression of a gene or target gene when the siRNA expressed in the same cell as the gene or target gene. In the context of this disclosure, the term "siRNA" includes miRNA. "siRNA" thus refers to the double stranded RNA formed by the complementary strands. The complementary portions of the siRNA that hybridize to form the double stranded molecule typically have substantial or complete identity. siRNA may therefore refer to a nucleic acid that has substantial or complete identity to a target gene and forms a double stranded siRNA. The sequence of the siRNA can correspond to the full length target gene, or a subsequence thereof. Typically, the siRNA is at least about 15-50 nucleotides in length (*e.g.,* each complementary sequence of the double stranded siRNA is 15-50 nucleotides in length, and the double stranded siRNA is about 15-50 base pairs in length, preferable about preferably about 20-30 base nucleotides, preferably about 20-25 nucleotides in length, *e.g.,* 20, 21, 22, 23, 24, 25, 26, 27, 28, 29, or 30 nucleotides in length.

The term "shRNA" refers generally to an siRNA that is introduced into a cell as part of a larger DNA construct. Typically, such constructs allow stable expression of the siRNA in cells after introduction, e.g., by integration of the construct into the host genome.

An "antisense" oligonucleotide or polynucleotide is a nucleotide sequence that is substantially complementary to a target polynucleotide or a portion thereof and has the ability to specifically hybridize to the target polynucleotide.

Ribozymes are enzymatic RNA molecules capable of catalyzing specific cleavage of RNA. The composition of ribozyme molecules preferably includes one or more sequences complementary to a target mRNA, and the well known catalytic sequence responsible for mRNA cleavage or a functionally equivalent sequence *(see, e.g.,* U.S. Pat. No. 5,093,246). Ribozyme molecules designed to catalytically cleave target mRNA transcripts can also be used to prevent translation of subject target mRNAs.

Amino acids can be referred to herein by either their commonly known three letter symbols or by the one-letter symbols recommended by the IUPAC-IUB Biochemical Nomenclature Commission. Nucleotides, likewise, can be referred to by their commonly accepted single-letter codes.

"Conservatively modified variants" as used herein applies to amino acid sequences. One of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

"Inhibitors," "activators," and "modulators" of expression or of activity are used to refer to inhibitory, activating, or modulating molecules, respectively, identified using *in vitro* and *in vivo* assays for expression or activity of a described target protein (or encoding polynucleotide), *e.g*., ligands, agonists, antagonists, and their homologs and mimetics. The term "modulator" includes inhibitors and activators. Inhibitors are agents that, *e.g.*, inhibit expression or bind to, partially or totally block stimulation or protease inhibitor activity, decrease, prevent, delay activation, inactivate, desensitize, or down regulate the activity of the described target protein, *e.g.,* antagonists. Activators are agents that, *e.g.,* induce or activate the expression of a described target protein or bind to, stimulate, increase, open, activate, facilitate, enhance activation or protease inhibitor activity, sensitize or up regulate the activity of described target protein (or encoding polynucleotide), *e.g*., agonists. Modulators include naturally occurring and synthetic ligands, antagonists and agonists (e.g., small chemical molecules, antibodies and the like that function as either agonists or antagonists). Such assays for inhibitors and activators include, *e.g*., applying putative modulator compounds to cells expressing the described target protein and then determining the functional effects on the described target protein activity, as described above. Samples or assays comprising described target protein that are treated with a potential activator, inhibitor, or modulator are compared to control samples without the inhibitor, activator, or modulator to examine the extent of effect. Control samples (untreated with modulators) are assigned a relative activity value of 100%. Inhibition of a described target protein is achieved when the activity value relative to the control is about 80%, optionally 50% or 25, 10%, 5% or 1%. Activation of the described target protein is achieved when the activity value relative to the control is 110%, optionally 150%, optionally 200, 300%, 400%, 500%, or 1000-3000% or more higher.

An "Oct polypeptide" refers to any of the naturally-occurring members of Octamer family of transcription factors, or variants thereof that maintain transcription factor activity, similar (within at least 50%, 80%, or 90% activity) compared to the closest related naturally occurring family member, or polypeptides comprising at least the DNA-binding domain of the naturally occurring family member, and can further comprise a transcriptional activation domain. Exemplary Oct polypeptides include, Oct-1, Oct-2, Oct-3/4, Oct-6, Oct-7, Oct-8, Oct-9, and Oct-11. *e.g.* Oct3/4 (referred to herein as "Oct4") contains the POU domain, a 150 amino acid sequence conserved among Pit-1, Oct-1, Oct-2, and uric-86. *See,* Ryan, A.K. & Rosenfeld, M.G. Genes Dev. 11, 1207-1225 (1997). In some embodiments, variants have at least 85%, 90%, or 95% amino acid sequence identity across their whole sequence compared to a naturally occurring Oct polypeptide family member such as to those listed above or such as listed in Genbank accession number NP_002692.2 (human Oct4) or NP_038661.1 (mouse Oct4). Oct polypeptides *(e.g.,* Oct3/4) can be from human, mouse, rat, bovine, porcine, or other animals. Generally, the same species of protein will be used with the species of cells being manipulated.

A "Klf polypeptide" refers to any of the naturally-occurring members of the family of Krüppel-like factors (Klfs), zinc-finger proteins that contain amino acid sequences similar to those of the Drosophila embryonic pattern regulator Krüppel, or variants of the naturally-occurring members that maintain transcription factor activity similar (within at least 50% , 80%, or 90% activity) compared to the closest related naturally occurring family member , or polypeptides comprising at least the DNA-binding domain of the naturally occurring family member, and can further comprise a transcriptional activation domain. *See,* Dang, D.T., Pevsner, J. & Yang, V.W.. Cell Biol. 32, 1103-1121 (2000). Exemplary Klf family members include, Klf1, Klf2, Klf3, Klf-4, Klf5, Klf6, Klf7, Klf8, Klf9, Klf10, Klf11, Klf12, Klf13, Klf14, Klf15, Klf16, and Klf17. Klf2 and Klf-4 were found to be factors capable of generating iPS cells in mice, and related genes Klf1 and Klf5 did as well, although with reduced efficiency. *See,* Nakagawa, et al, Nature Biotechnology 26:101 - 106 (2007). In some embodiments, variants have at least 85%, 90%, or 95% amino acid sequence identity across their whole sequence compared to a naturally occurring Klf polypeptide family member such as to those listed above or such as listed in Genbank accession number CAX16088 (mouse Klf4) or CAX14962 (human Klf4). Klf polypeptides (e.g., Klf1, Klf4, and Klf5) can be from human, mouse, rat, bovine, porcine, or other animals. Generally, the same species of protein will be used with the species of cells being manipulated. To the extent a Klf polypeptide is described herein, it can be replaced with an estrogen-related receptor beta (Essrb) polypeptide. Thus, it is intended that for each Klf polypeptide embodiment described herein, a corresponding embodiment using Essrb in the place of a Klf4 polypeptide is equally described.

A "Myc polypeptide" refers any of the naturally-occurring members of the Myc family *(see, e.g.,* Adhikary, S. & Eilers, M. Nat. Rev. Mol. Cell Biol. 6:635-645 (2005)), or variants thereof that maintain transcription factor activity similar (within at least 50%, 80%, or 90% activity) compared to the closest related naturally occurring family member , or polypeptides comprising at least the DNA-binding domain of the naturally occurring family member, and can further comprise a transcriptional activation domain. Exemplary Myc polypeptides include, *e.g.,* c-Myc, N-Myc and L-Myc. In some embodiments, variants have at least 85%, 90%, or 95% amino acid sequence identity across their whole sequence compared to a naturally occurring Myc polypeptide family member, such as to those listed above or such as listed in Genbank accession number CAA25015 (human Myc). Myc polypeptides (e.g., c-Myc) can be from human, mouse, rat, bovine, porcine, or other animals. Generally, the same species of protein will be used with the species of cells being manipulated.

A "Sox polypeptide" refers to any of the naturally-occurring members of the SRY-related HMG-box (Sox) transcription factors, characterized by the presence of the high-mobility group (HMG) domain, or variants thereof that maintain transcription factor activity similar (within at least 50%, 80%, or 90% activity) compared to the closest related naturally occurring family member , or polypeptides comprising at least the DNA-binding domain of the naturally occurring family member, and can further comprise a transcriptional activation domain. *See, e.g.,* Dang, D.T., et al., Int. J. Biochem. Cell Biol. 32:1103-1121 (2000). Exemplary Sox polypeptides include, e.g., Sox1, Sox-2, Sox3, Sox4, Sox5, Sox6, Sox7, Sox8, Sox9, Sox10, Sox11, Sox12, Sox13, Sox14, Sox15, Sox17, Sox18, Sox-21, and Sox30. Sox1 has been shown to yield iPS cells with a similar efficiency as Sox2, and genes Sox3, Sox15, and Sox18 have also been shown to generate iPS cells, although with somewhat less efficiency than Sox2. *See,* Nakagawa, et al., Nature Biotechnology 26:101 - 106 (2007). In some embodiments, variants have at least 85%, 90%, or 95% amino acid sequence identity across their whole sequence compared to a naturally occurring Sox polypeptide family member such as to those listed above or such as listed in Genbank accession number CAA83435 (human Sox2). Sox polypeptides (*e.g.,* Sox1, Sox2, Sox3, Sox15, or Sox18) can be from human, mouse, rat, bovine, porcine, or other animals. Generally, the same species of protein will be used with the species of cells being manipulated.

### DETAILED DESCRIPTION OF THE INVENTION

### Introduction

The invention provides methods of increasing the efficiency of obtaining pluripotent stem cells, the method comprising expressing a Δ133p53 in cells that are being re-programmed to obtain pluripotent cells. In a typical embodiment described herein, the somatic cells to be re-programmed are partially- or fully-differentiated cells *(e.g.,* blood cells, dermal cells, fibroblasts, epithelial cells, keratinocytes, and the like). Cells to be re-programmed express one or more pluripotency induction factors, *e.g.,* Oct3/4, Sox2, Klf4, c-Myc, Lin28, or Nanog. Cells can be induced for re-programming using a variety of methods, including contacting the cells with a chemical compound to increase the expression of induction forms and/or by enhancing expression by expressing an exogenous induction factor in the cell.

In the methods of the invention, increased number of iPS cells are obtained from the starting population of cells to be re-programmed by increasing the level of expression of Δ133p53. Typically, Δ133p53 expression is increased by introducing a gene encoding Δ133p53 into the cells. The invention is described in further detail herein below.

### Methods for isolating nucleotide sequences encoding Δ133p53

In general, the nucleic acid sequences encoding Δ133p53 or p53β and related nucleic acid sequence homologues can be cloned from cDNA libraries or are typically isolated using amplification techniques with oligonucleotide primers.

Advantageously, the cloning of Δ133p53 or p53β or other p53 isoforms can employ the use of synthetic oligonucleotide primers and amplification of an RNA or DNA template *(see* U.S. Patents 4,683,195 and 4,683,202; *PCR Protocols: A Guide to Methods and Applications* (Innis *et al,* eds, 1990)). Methods such as polymerase chain reaction (PCR) and ligase chain reaction (LCR) can be used to amplify nucleic acid sequences of Δ133p53 or p53β directly from mRNA, from cDNA, from genomic libraries or cDNA libraries. Degenerate oligonucleotides can be designed to amplify Δ133p53 or p53β homologues for other species using known sequences. Restriction endonuclease sites can be incorporated into the primers. Genes amplified by the PCR reaction can be purified from agarose gels and cloned into an appropriate vector.

The nucleic acids encoding Δ133p53 or p53β or other p53 isoforms are typically cloned into intermediate vectors before transformation into prokaryotic or eukaryotic cells for replication and/or expression. These intermediate vectors are typically prokaryote vectors, *e.g.*, plasmids, or shuttle vectors. The isolated nucleic acids encoding Δ133p53 or p53β or other p53 isoforms comprise nucleic acid sequences these proteins and subsequences, interspecies homologues, alleles and polymorphic variants thereof. The introduction of Δ133p53 into cell populations that undergo re-programming into iPS cells is discussed in greater detail hereinbelow.

### Pluripotent Stem Cells

Any animal can be used as a source of somatic cells to obtain iPS in the methods of the invention. Typically, the somatic cells are human cells, although cells from other animals can be used. Thus, for example, in some embodiments, the cells are mammalian cells. Exemplary mammalian cells include, but are not limited to, human cells; non-human primate cells, *e.g.,* rat, mouse, porcine, bovine, ovine, canine, and feline cells; and primate cells *(e.g.,* rhesus monkeys, chimpanzee, macaques, etc.).

Methods of generating human iPS cells are known in the art, and a wide range of methods can be used to generate iPS cells (see, *e.g.,* Takahashi and Yamanaka Cell 126:663-676, 2006; Blelloch et al, Cell Stem Cell 1:245-247, 2007; Yamanaka et al., Nature 448:313-7, 2007; Wernig et al., Nature 448:318-24, 2007; Maherali et al., Cell Stem Cell 1 :55-70, 2007; Maherali & Hochedlinger Cell Stem Cell 3:595-605, 2008; Park et al., Cell 134: 1-10, 2008; Dimos et al., Science 321:1218-1221, 2008; Stadtfeld et al., Science 322:945- 949, 2008; Stadtfeld et al., Cell Stem Cell 2:230-240, 2008; Okita et al, Science 322:949-953, 2008. Methods for inducing multipotent and pluripotent stem cell lines are further disclosed in U.S. Patent Application Publication No. 20090191159 and WO/2010/059806. Other methods include introducing modified RNA molecules that encode transcription factors that induce iPS cells, *e.g.,* KLF4, C-MYC, OCT4, and SOX2 (Warren et al, Cell Stem Cell 7:1-13, 2010; published online September 30, 2010.) Thus, increasing Δ133p53 expression can be performed in conjunction with any other iPS induction method, including serial protein transduction with recombinant proteins incorporates cell-penetrating peptides moieties (Kim et al, Cell Stem Cell 4:472-476, 2009; Zhou et al, Cell Stem Cell 4:381-384, 2009); through repeated application of transient plasmid, episomal, and adenovirus vectors *(see, e.g.,* Okita et al, Science, 2008, *supra;* Stadtfeld et al, Science 2008, *supra;* Woltjen *et al,* 2009, *supra;* Yu et al, Science 324:797-801, 2009); transgene delivery using the Sendai virus (Fusake et al, Proc. Jpn. Acad. Ser. B, Phys. Biol. Sci 85:348-362,2009) as well as methods using retroviral vectors and other methods to induce transcription factors.

Pluripotency can be induced, for example, by introduction of transcription factors or using agents that otherwise induce or mimic expression of certain transcription factors. In some embodiments, one or more of the following transcription factors are expressed endogenously or recombinantly (*e.g.*, by introduction of heterologous expression cassettes expressing one or more transcription factors). Illustrative technologies for induction of pluripotency include, but are not limited to, introduction of at least one or more expression cassette for expression of at least one of Oct3/4, Sox2, c-Myc, and Klf4 (*see, e.g.,* Takahashi, Cell 131(5):861-872, 2007; Cell Stem Cell 2, 10-12, 2008), optionally with one or more small molecules, including but not limited to, an agent that inhibits H3K9 methylation, *e.g.*, a G9a histone methyltransferase such as BIX01294 *(see, e.g.,* Kubicek, et al., Mol. Cell473-481, 2007) or a small molecule that inhibits TGFβ signaling. Thus, in performing the methods of the invention, the cells, typically adult somatic cells such as fibroblasts, in which Δ133p53 expression is enhanced may be treated with a variety of agents for re-programming, including epigenetic re-programming agents.

The pluripotent cells obtained using the methods of the invention can be characterized by several criteria. In addition to the gene expression, methylation, and *in vitro* and *in vivo* characteristics described herein, the pluripotent cells of the invention will maintain pluripotency over at least one *(e.g.,* 1, 2, 3, 4, 5, 10, 20, etc.) cell divisions. The cells may be maintained in media that comprises particular growth factors, *e.g.*, leukemia inhibitory factor (LIF) and bone morphogenic protein (BMP); or, under conditions that inhibit the TGFβ and activin signaling pathway, inhibition of the MAPK signaling pathway, and optionally inhibition of the FGF pathway. Typically, human iPS cells differentiate when treated with MEK, FGFR and/or ALK4/5/7 inhibitors (Brons et al., Nature 448, 191-195, 2007; Li et al., Differentiation 75, 299-307, 2007; Peerani et al., EMBO J 26, 4744-4755, 2007; Tesar et al., Nature 448, 196-199, 2007).

Various markers can also be used to characterize iPS cells obtained in accordance with the method of the invention. These include alkaline phosphatase, α-fetoprotein (AFP), BMP, and other markers, such as SSEA-3, SSEA-4, TRA-1-60, TRA-1-81, TRA-2-49/6E, Nanog. GDF3, REX1, FGF4, ESG1, DPPA2, DPPA4, and hTERT.

In some embodiments, somatic cells that are to be induced to obtain iPS cells are also manipulated, e.g., by transfecting with appropriate expression vectors that encode the desired protein, to express at least one of Oct3/4, Sox2, c-Myc, and Klf4. In typical embodiments, the somatic cells expressOct3/4, Sox2, and Klf4. In some embodiments, the somatic cells express Oct3/4, Sox2, Klf, and c-myc. In some embodiments, the cells to be induced, *e.g.*, somatic cells, such as fibroblasts and the like, express one or more of Oct3/4, Sox2, Klf, and c-myc prior to expression of Δ133p53. In some embodiments, the cells to be induced are transfected with expression constructs that express Δ133p53 at the same time as transfection with expression vectors that express one or more Oct3/4, Sox2, Klf, and c-myc. In further embodiments, a vector that expresses Δ133p53 is introduced into the cell population to be induced prior to introduction of nucleic acids that encode one or more of Oct3/4, Sox2, Klf, and c-myc.

Methods known in the art can be used to characterize the cells of the invention. Gene expression levels, *e.g.,* of Δ133p53, a transcription factor, or other protein indicative of reprogramming, can be detected by, *e.g.,* real-time PCR or real-time RT-PCR *(e.g.,* to detect mRNA), and/or by western blot or other protein detection technique. (See for example, references such as WO/2010/033991 and references cited therein, which describe methods of detecting somatic cell reprogramming.)

In some instances, the efficiency of obtaining iPS from a population of cells that is being re-programmed, e.g., differentiated or partially differentiated somatic cells, can be increased, e.g., by at least 1.5-fold, 2-fold, or 3-fold, or more, by inhibiting the level of expression of p53β. Such methods can employ, *e.g.*, nucleic acids such as inhibitory RNA molecules that specifically target p53β. Such methods can be used in conjunction with methods of the invention that increase Δ133p53 expression or may be employed independently on cells as described herein that are being re-programmed.

In some embodiments, the somatic cells that are re-programmed are adult somatic cells. Suitable mammalian somatic cells can also include, but are not limited to, Sertoli cells, endothelial cells, granulosa epithelial, neurons, pancreatic islet cells, epidermal cells, epithelial cells, hepatocytes, hair follicle cells, keratinocytes, hematopoietic cells, melanocytes, chondrocytes, lymphocytes (B and T lymphocytes), erythrocytes, macrophages, monocytes, mononuclear cells, cardiac muscle cells, and other muscle cells, etc. In some embodiments, the differentiated somatic cell to be reprogrammed is a fibroblast, e.g., an adult dermal fibroblast, In some embodiments, the differentiated cell is an embryonic fibroblast. In some embodiments, the differentiated cell is a cell of hematopoietic lineage. In some embodiments, the differentiated cell is derived from peripheral blood. In some embodiments, the somatic cells are adult stem cells, e.g., cells that are capable of giving rise to all the cell types of a particular tissue. Illustrative adult stem cells include hematopoietic stem cells, neural stem cells, cardiac stem cells, *e.g.*, cardiosphere derived cells, and mesenchymal stem cells.

### Cancer stem cells

In another aspect, the disclosure provides methods of inhibiting cancer stem cell proliferation by decreasing the level of expression of Δ133p53. Thus, cancer stems cells can be targeted for suppressing of Δ133p53, thereby inhibiting proliferation of the cells. Suppression can be performed, e.g., using inhibitory RNA molecule that target Δ133p53.

In a further aspect, the disclosure provides methods of inhibiting cancer stem cells proliferation by increasing the level of expression of p53β, *e.g.,* by using an expression construct. One of skill understands that expression of p53β can be performed using the same techniques for constructing vectors and introducing nucleic acids into the cells as those described for Δ133p53.

Cancer stem cells (CSCs) can be identified using methods well known in the art. The most common method of identifying CSCs in a tumor is through the identification of cell surface markers that also identify normal stem cells in the tissue of origin. For instance, leukemic stem cells (LSCs) express the CD34 surface marker and lack the CD38 surface antigen, as is the case for normal (i.e., non-leukemic) hematopoietic stem cells. Prostate CSCs are positive for the CD 133 and CD44 surface antigens (CD133+CD44+). Breast cancer stem cells are often CD44+/CD24-. CSCs identified by cell surface marker expression can be purified by methods such as fluorescence-activated cell sorting (FACS).

Besides cell surface marker expression, functional assays are also frequently used to identify and isolate CSCs. Normal stem cells and cancer stem cells preferentially express ATP-binding cassette (ABC) transporters, membrane protein complexes that enable the cells to resist cytotoxic drugs by actively transporting toxic compounds out of the cell. When cells are stained with the Hoechst 33342 fluorescent dye, ABC transporters in the stem cells actively pump the dye out of the cell. Stem cells thus appear as a "Hoechst-low" population when analyzed by FACS; whereas non-stem cells are more highly fluorescent. This selection technique has been successfully applied to identify and isolate stem cells and CSCs from a variety of tissues, including the blood, brain, and breast.

### Expression of Δ133p53 in cells to be re-programmed as iPS cells

Expression of Δ133p53 (and of other proteins such as transcription factors that induce iPS) is performed using techniques well known in the art. Thus, the invention relies on routine techniques in the field of recombinant genetics. Basic texts disclosing the general methods of use in this invention include Sambrook and Russell (2001) Molecular Cloning: A laboratory manual 3rd ed. Cold Spring Harbor Laboratory Press; and Current Protocols in Molecular Biology (2010) John Wiley and Sons.

Any number of vectors may be employed, including plasmid and viral vectors. It will be appreciated that where two or more proteins are to be expressed in a cell, *e.g.,* when a nucleic acid encoding Δ133p53 and/or one or more transcription factors to induce iPS are expressed in a cell, one or multiple expression cassettes can be used. For example, where one expression cassette is to express multiple polypeptides, a polycistronic expression cassette can be used.

### Plasmid Vectors

In certain embodiments, a plasmid vector is contemplated for use to transform a host cell. In general, plasmid vectors containing replicon and control sequences which are derived from species compatible with the host cell are used in connection with these hosts. Expression vectors containing regulatory elements from eukaryotic viruses are typically used in eukaryotic expression vectors, e.g., SV40 vectors, papilloma virus vectors, and vectors derived from Epstein-Barr virus. Other exemplary eukaryotic vectors include pMSG, pAV009/A+, pMTO10/A+, pMAMneo-5, baculovirus pDSVE, and any other vector allowing expression of proteins under the direction of the SV40 early promoter, SV40 later promoter, metallothionine promoter, murine mammary tumor virus promoter, Rous sarcoma virus promoter, polyhedrin promoter, or other promoters shown effective for expression in eukaryotic cells. In some embodiments, the promoter used to express a Δ133p53 nucleic acid and/or a transcription factor for inducing iPS may be an inducible promoter, such as an antiobiotic-based promoter (e.g., tet) promoter. Such promoters are known in the art.

### Viral Vectors

The ability of certain viruses to infect cells or enter cells via receptor-mediated endocytosis, and to integrate into host cell genome and express viral genes stably and efficiently have made them attractive candidates for the transfer of foreign nucleic acids into cells (e.g., mammalian cells). Non-limiting examples of virus vectors that may be used to deliver a nucleic acid are described below. Vectors for use in the invention include adenoviral vectors, adeno-associated viral vectors, retroviral vectors, such as lentiviral vectors, poxvirus vectors, herpes virus vectors and the like.

A nucleic acid to be delivered to target cells may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. his modification can permit the specific infection of hepatocytes via sialoglycoprotein receptors.

Another approach to targeting of recombinant retroviruses was designed in which biotinylated antibodies against a retroviral envelope protein and against a specific cell receptor were used. The antibodies were coupled via the biotin components by using streptavidin (Roux et al., Proc. Nat'l Acad. Sci. USA, 86:9079-9083, 1989). Using antibodies against major histocompatibility complex class I and class II antigens, they demonstrated the infection of a variety of human cells that bore those surface antigens with an ecotropic virus in vitro (Roux et al., 1989).

In some embodiments, the Δ133p53 nucleic acid that is introduced into the cells to be reprogrammed is an RNA molecule. Such an RNA molecule can be prepared, *e.g.*, using *in vitro* transcription (see, *e.g.,* Warren et al., Cell Stem Cell 7:1-13, 2010, published on line September 30,2010).

### Vector Delivery and Cell Transformation

Suitable methods for nucleic acid delivery for transformation of a cell, a tissue or an organism for use with the current invention are believed to include virtually any method by which a nucleic acid (e.g., DNA) can be introduced into a cell, a tissue or an organism, as described herein or as would be known to one of ordinary skill in the art. Such methods include, but are not limited to, direct delivery of DNA such as by ex vivo transfection (Wilson et al., Science, 244:1344-1346, 1989, Nabel and Baltimore, Nature 326:711-713, 1987), optionally with Fugene6 (Roche) or Lipofectamine (Invitrogen), by injection (U.S. Pat. Nos. 5,994,624, 5,981,274, 5,945,100, 5,780,448, 5,736,524, 5,702,932, 5,656,610, 5,589,466 and 5,580,859), including microinjection and gene gun injection (Harland and Weintraub, J. Cell Biol., 101:1094-1099,1985; U.S. Pat. No. 5,789,215,); by electroporation (U.S. Pat. No. 5,384,253; Tur-Kaspa et al., Mol. Cell Biol., 6:716-718, 1986; Potter et al., Proc. Nat'l Acad. Sci. USA, 81:7161-7165, 1984); by calcium phosphate precipitation (Graham and Van Der Eb, Virology, 52:456-467,1973; Chen and Okayama, Mol. Cell Biol., 7(8):2745-2752, 1987; Rippe et al., Mol. Cell Biol., 10:689-695, 1990); by using DEAE-dextran followed by polyethylene glycol (Gopal, Mol. Cell Biol., 5:1188-1190, 1985); by direct sonic loading (Fechheimer et al., Proc. Nat'l Acad. Sci. USA, 84:8463-8467, 1987); by liposome mediated transfection (Nicolau and Sene, Biochim. Biophys. Acta, 721:185-190, 1982; Fraley et al., Proc. Nat'l Acad. Sci. USA, 76:3348-3352, 1979; Nicolau et al., Methods Enzymol., 149:157-176, 1987; Wong et al., Gene, 10:87-94, 1980; Kaneda et al., Science, 243:375-378, 1989; Kato et al., J Biol. Chem., 266:3361-3364, 1991) and receptor-mediated transfection (Wu and Wu, Biochemistry, 27:887-892, 1988; Wu and Wu, J. Biol. Chem., 262:4429-4432, 1987). Nucleic acids can also be introduced into cells using methods as described below in the section describing inhibitory nucleic acids. Further, any combination of such methods may be employed.

### Culturing of cells

Cells that are undergoing re-programming to obtain iPS cells, can be cultured according to any method known in the art. Various techniques are known (see, e.g., WO/2010/059775; WO/2010/077955. WO/2010/002846, Warren *et al,* 2010, *supra,* and references cited therein). Culture media can include any other component of culture media as known in the art. In some embodiments, cells are cultured under low oxygen conditions. The cultures can include serum or can be serum-free.

In some embodiments, the cells are cultured in contact with feeder cells. Illustrative feeder cells include, but are not limited to fibroblast cells, *e.g.*, mouse embryonic fibroblast (MEF) cells. Methods of culturing cells on feeder cells is known in the art.

In some embodiments, the cells are cultured in the absence of feeder cells. Cells, for example, can be attached directly to a solid culture surface *(e.g.,* a culture plate), *e.g.,* via a molecular tether. Examples of molecular tethers include, but are not limited to, matrigel, an extracellular matrix (ECM), ECM analogs, laminin, fibronectin, or collagen. Methods for initial attachment of the tethers to the solid surface are known in the art.

### Differentiation of iPS cells obtained in accordance with the methods of the invention

The iPS cells obtained using the methods of the invention can be used for any number of applications, including clinical and research applications. In some applications, the cells may be differentiated cell types that are used to assess the effects of drugs on that cell type. In some applications, the cells may be employed *in vivo.* The iPS cells may be differentiated into any number of cell types, such as neurons, cardiac muscle cells, liver hepatocytes and lung respiratory epithelial cells.

When differentiating the iPS cells of the invention, it is typically desirable to decrease Δ133p53 expression. Thus, in some embodiment, Δ133p53 is expressed under the control of an inducible promoter. The inducing agents can then be moved when it is desired to initiate iPS differentiation. In some embodiments, the Δ133p53 expression is disrupted using a recombination-based system. Thus, in certain instances, Δ133p53 may be expressed using a genetic construct that has a lox recombination site. Upon expression of a cre recombinase, the Δ133p53 transgene can then be disrupted to suppress expression. Thus, iPS cells can be derived via excisable lentiviral and transposon vectors (*see, e.g.,* Chang et al., Stem Cells 27:1042-1049, 2009; Kaji et al., Nature 458:771-775, 2009,.)

In further embodiments, expression of Δ133p53 can be performed using further genetic manipulation of the cells, *e.g.,* by targeting the transgene with an inhibitory nucleic acid.

In other aspects, described herein is a method of facilitating differentiation of iPS or embryonic stem cells, the method comprising increasing the expression level of p53β. Such methods can be performed in conjunction with decreasing Δ133p53 expression or on cells that have not been previously subject to treatment to increase Δ133p53 expression.

### Inhibition of p53 isoforms using nucleic acids

In some aspects of the disclosure, it is desirable to disrupt endogenous expression of Δ133p53 in a stem cell such as a cancer stem cells, or to disrupt endogenous expression of p53β. Inhibitory nucleic acids to Δ133p53 and p53β such as siRNA, shRNA, ribozymes, or antisense molecules, can be synthesized and introduced into cells using methods known in the art. Molecules can be synthesized chemically or enzymatically *in vitro* (Micura, Agnes Chem. Int. Ed. Emgl. 41: 2265-9 (2002); Paddison et al., Proc. Natl. Acad. Sci. USA, 99: 1443-8 2002) or endogenously expressed inside the cells in the form of shRNAs (Yu et al., Proc. Natl. Acad. Sci. USA, 99: 6047-52 (2002); McManus et al, RNA 8, 842-50 (2002)). Plasmid-based expression systems using RNA polymerase III U6 or H1, or RNA polymerase II U1, small nuclear RNA promoters, have been used for endogenous expression of shRNAs (Brummelkamp et al, Science, 296: 550-3 (2002); Sui et al., Proc. Natl. Acad. Sci. USA, 99: 5515-20 (2002); Novarino et al., J. Neurosci., 24: 5322-30 (2004)). Synthetic siRNAs can be delivered by electroporation or by using lipophilic agents (McManus et al., RNA 8, 842-50 (2002); Kishida et al., J. Gene Med., 6: 105-10 (2004)). Alternatively, plasmid systems can be used to stably express small hairpin RNAs for the suppression of target genes (Dykxhoorn et al., Nat. Rev. Mol. Biol., 4: 457-67 (2003)). Various viral delivery systems have been developed to deliver shRNA-expressing cassettes into cells that are difficult to transfect (Brummelkamp et al., Cancer Cell, 2: 243-7 (2002); Rubinson et al., Nat. Genet., 33: 401-6 2003). Furthermore, siRNAs can also be delivered into live animals. (Hasuwa et al., FEBS Lett., 532, 227-30 (2002); Carmell et al., Nat. Struct. Biol., 10: 91-2 (2003); Kobayashi et al., J. Pharmacol. Exp. Ther., 308: 688-93 (2004)).

Methods for the design of siRNA or shRNA target sequences have been described in the art. Among the factors to be considered include: siRNA target sequences should be specific to the gene of interest and have ∼20-50% GC content (Henshel et al, Nucl. Acids Res., 32: 113-20 (2004); G/C at the 5' end of the sense strand; A/U at the 5' end of the antisense strand; at least 5 A/U residues in the first 7 bases of the 5' terminal of the antisense strand; and no runs of more than 9 G/C residues (Ui-Tei et al, Nucl. Acids Res., 3: 936-48 (2004)). Additionally, primer design rules specific to the RNA polymerase will apply. For example, for RNA polymerase III, the polymerase that transcribes from the U6 promoter, the preferred target sequence is 5'-GN18-3'. Runs of 4 or more Ts (or As on the other strand) will serve as terminator sequences for RNA polymerase III and should be avoided. In addition, regions with a run of any single base should be avoided (Czauderna et al., Nucl. Acids Res., 31: 2705-16 (2003)). It has also been generally recommended that the mRNA target site be at least 50-200 bases downstream of the start codon (Sui et al., Proc. Natl. Acad. Sci. USA, 99: 5515-20 (2002); Elbashir et al., Methods, 26: 199-213 (2002); Duxbury and Whang, J. Surg. Res., 117: 339-44 (2004) to avoid regions in which regulatory proteins might bind. Additionally, a number of computer programs are available to aid in the design of suitable siRNA and shRNAs.

Ribozymes that cleave mRNA at site-specific recognition sequences can be used to destroy target mRNAs, particularly through the use of hammerhead ribozymes. Hammerhead ribozymes cleave mRNAs at locations dictated by flanking regions that form complementary base pairs with the target mRNA. Preferably, the target mRNA has the following sequence of two bases: 5'-UG-3'. The construction and production of hammerhead ribozymes is well known in the art.

Gene targeting ribozymes necessarily contain a hybridizing region complementary to two regions, each of at least 5 and preferably each 6, 7, 8, 9, 10, 11, 12, 13, 14, 15, 16, 17, 18, 19 or 20 contiguous nucleotides in length of a target mRNA, e.g., an mRNA targeting Δ133p53. In addition, ribozymes possess highly specific endoribonuclease activity, which autocatalytically cleaves the target sense mRNA.

With regard to antisense, siRNA or ribozyme oligonucleotides, phosphorothioate oligonucleotides can be used. Modifications of the phosphodiester linkage as well as of the heterocycle or the sugar may provide an increase in efficiency. Phophorothioate is used to modify the phosphodiester linkage. An N3'-P5' phosphoramidate linkage has been described as stabilizing oligonucleotides to nucleases and increasing the binding to RNA. Peptide nucleic acid (PNA) linkage is a complete replacement of the ribose and phosphodiester backbone and is stable to nucleases, increases the binding affinity to RNA, and does not allow cleavage by RNAse H. Its basic structure is also amenable to modifications that may allow its optimization as an antisense component. With respect to modifications of the heterocycle, certain heterocycle modifications have proven to augment antisense effects without interfering with RNAse H activity. An example of such modification is C-5 thiazole modification. Finally, modification of the sugar may also be considered. 2'-O-propyl and 2'-methoxyethoxy ribose modifications stabilize oligonucleotides to nucleases in cell culture and *in vivo.*

One of skill understands that inhibitory nucleic acids that target p53β can be obtained using the same principles and methods.

Inhibitory oligonucleotides can be delivered to a cell by direct transfection or transfection and expression via an expression vector. Appropriate expression vectors include mammalian expression vectors and viral vectors, into which has been cloned an inhibitory oligonucleotide with the appropriate regulatory sequences including a promoter to result in expression of the antisense RNA in a host cell. Suitable promoters can be constitutive or development-specific promoters. Transfection delivery can be achieved by liposomal transfection reagents, known in the art (*e.g.*, Xtreme transfection reagent, Roche, Alameda, CA; Lipofectamine formulations, Invitrogen, Carlsbad, CA). Delivery mediated by cationic liposomes, by retroviral vectors and direct delivery are efficient. Another possible delivery mode is targeting using antibody to cell surface markers for the target cells.

For transfection, a composition comprising one or more nucleic acid molecules (within or without vectors) can comprise a delivery vehicle, including liposomes, for administration to a subject, carriers and diluents and their salts, and/or can be present in pharmaceutically acceptable formulations. Methods for the delivery of nucleic acid molecules are described, for example, in Gilmore, et al., Curr Drug Delivery (2006) 3:147-5 and Patil, et al., AAPS Journal (2005) 7:E61 - E77. Delivery of siRNA molecules is also described in several U.S. Patent Publications, including for example, 2006/0019912; 2006/0014289; 2005/0239687; 2005/0222064; and 2004/0204377. Nucleic acid molecules can be administered to cells by a variety of methods known to those of skill in the art, including, but not restricted to, encapsulation in liposomes, by iontophoresis, by electroporation, or by incorporation into other vehicles, including biodegradable polymers, hydrogels, cyclodextrins (*see, for example* Gonzalez et al., 1999, Bioconjugate Chem., 10, 1068-1074; Wang et al, International PCT publication Nos. WO 03/47518 and WO 03/46185), poly(lactic-co-glycolic)acid (PLGA) and PLCA microspheres *(see* for example U.S. Pat. No. 6,447,796 and US Patent Application Publication No. 2002/130430), biodegradable nanocapsules, and bioadhesive microspheres, or by proteinaceous vectors (O'Hare and Normand, International PCT Publication No. WO 00/53722). Nucleic acid molecules can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives.

Examples of liposomal transfection reagents include, for example: CellFectin, 1:1.5 (M/M) liposome formulation of the cationic lipid N,NI,NII,NIII-tetramethyl-N,NI,NII,NIII-tetrapalmit-y-spermine and dioleoyl phosphatidylethanolamine (DOPE) (GIBCO BRL); Cytofectin GSV, 2:1 (M/M) liposome formulation of a cationic lipid and DOPE (Glen Research); DOTAP (N-[1-(2,3-dioleoyloxy)-N,N,N-tri-methyl-ammoniummethylsulfate) (Boehringer Manheim); Lipofectamine, 3:1 (M/M) liposome formulation of the polycationic lipid DOSPA and the neutral lipid DOPE (GIBCO BRL); and (5) siPORT (Ambion); HiPerfect (Qiagen); Xtreme GENE (Roche); RNAicarrier (Epoch Biolabs) and TransPass (New England Biolabs).

In some instances, antisense, siRNA, or ribozyme sequences are delivered into the cell via a mammalian expression vector. For example, mammalian expression vectors suitable for siRNA expression are commercially available, for example, from Ambion (*e.g.*, pSilencer vectors), Austin, TX; Promega (*e.g.*, GeneClip, siSTRIKE, SiLentGene), Madison, WI; Invitrogen, Carlsbad, CA; InvivoGen, San Diego, CA; and Imgenex, San Diego, CA. Typically, expression vectors for transcribing siRNA molecules will have a U6 promoter.

In some instances, antisense, siRNA, or ribozyme sequences are delivered into cells via a viral expression vector. Viral vectors suitable for delivering such molecules to cells include adenoviral vectors, adeno-associated vectors, and retroviral vectors (including lentiviral vectors). For example, viral vectors developed for delivering and expressing siRNA oligonucleotides are commercially available from, for example, GeneDetect, Bradenton, FL; Ambion, Austin, TX; Invitrogen, Carlsbad, CA; Open BioSystems, Huntsville, AL; and Imgenex, San Diego, CA.

### EXAMPLES

The following examples are offered to illustrate, but not to limit the claimed invention. Reference Example 1. Expression of Δ133p53 in embryonic stem cells

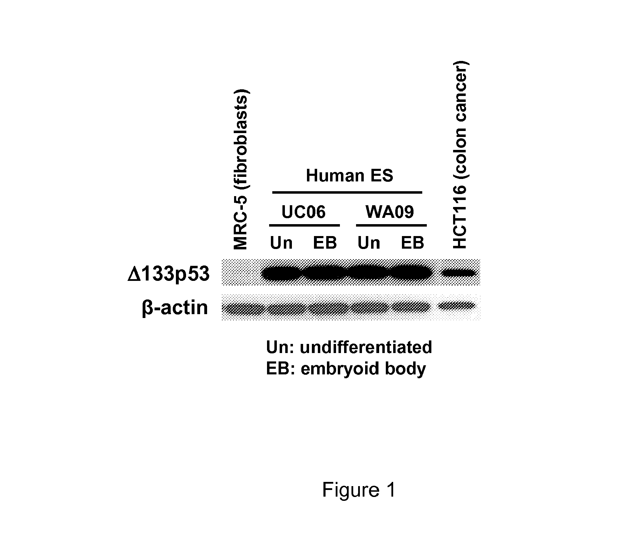

Human embryonic stem cells were evaluated for the expression of Δ133p53 protein. Protein lysates were isolated from human embryonic stem cells UC06 and WA09. The results (Figure 1) showed that undifferentiated and embryoid bodies expressed Δ133p53. Control fibroblasts showed little expression of Δ133p53. Colon cancer cells HCT116 showed lower levels of expression in comparison to the embryonic stem cells.

### Example 2. Expression of Δ133p53 in fibroblasts expressing reprogramming factors increase the number of iPS colonies.

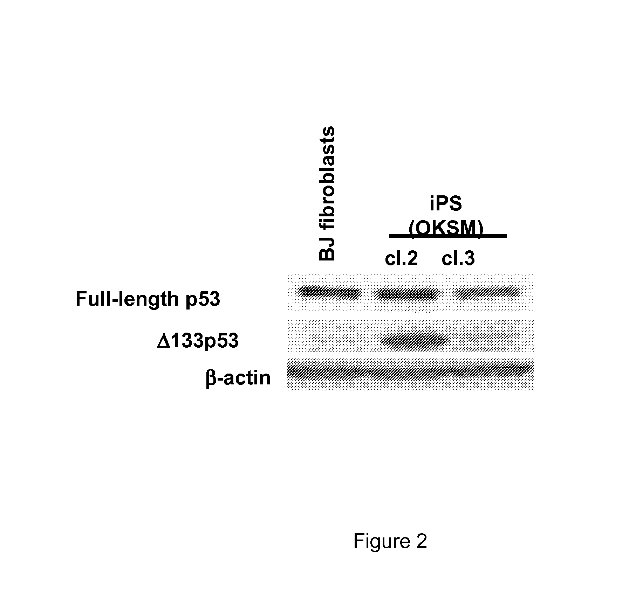

Figure 2 provides further data showing that either downregulation of endogenous full-length p53 or upregulation of endogenous Δ133p53 was associated with iPSC reprogramming. The iPS cell clones analyzed in this experiment were induced by four factors (OCT4, KLF4, SOX2, and c-Myc).

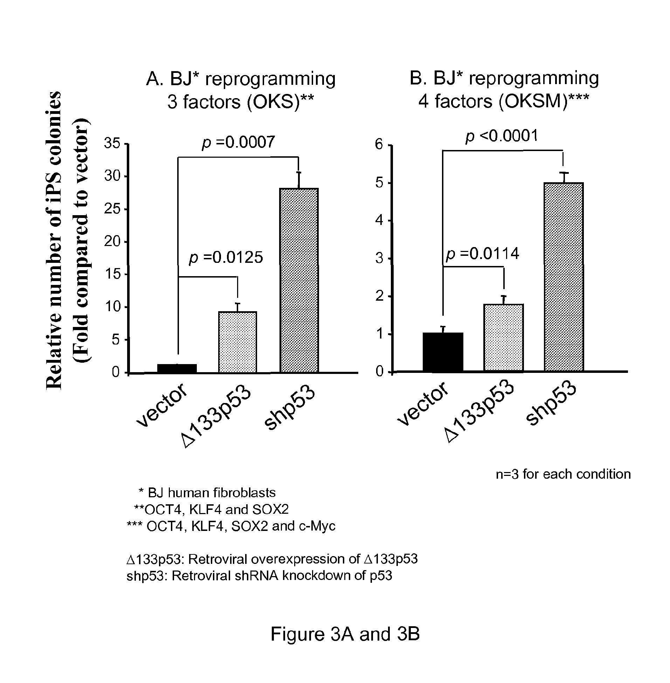

Fibroblasts cells were infected with retroviral vectors that expressed Δ133p53 or shRNA to knockdown p53, together with reprogramming factors to induce pluripotent stem cells (Figure 3A and 3B). In panel A, fibrobasts expressed three pluripotent stem cell inducing transcription factors, Oct4, Klf4, and Sox2. In panel B, fibroblasts expressed four transcription factors Oct4, Klf4, Sox2, and c-Myc. A vector control, a retrovirus construct expressing Δ133p53, or a positive control retroviral expression shRNA to knockdown p53 expression was introduced into the cells. Figure 3 demonstrates that Δ133p53 enhanced production of iPS cells relative to vector alone.

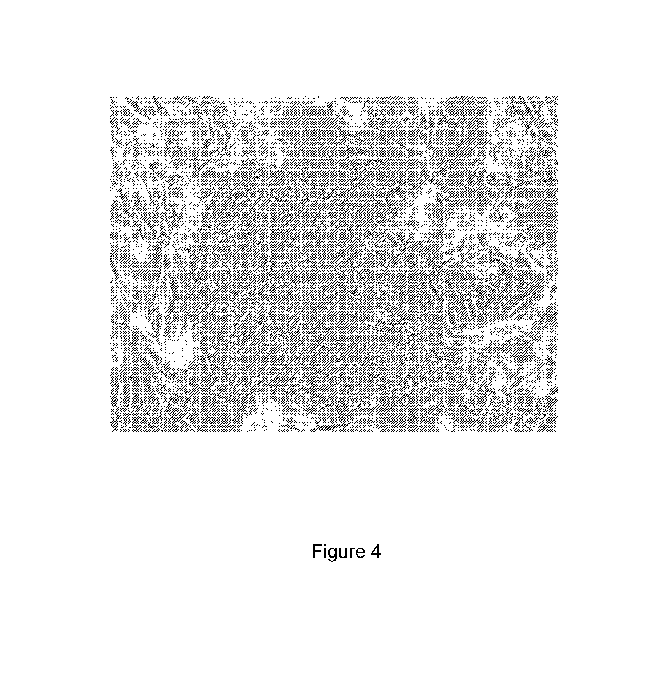

Figure 4 shows the cell morphology of iPS cells that were derived from BJ fibroblasts that were transduced with the retroviral vectors of 4 iPS factors (OKSM: OCT4, KLF4, SOX2 and c-Myc) and Δ133p53 overexpression.

### Methods

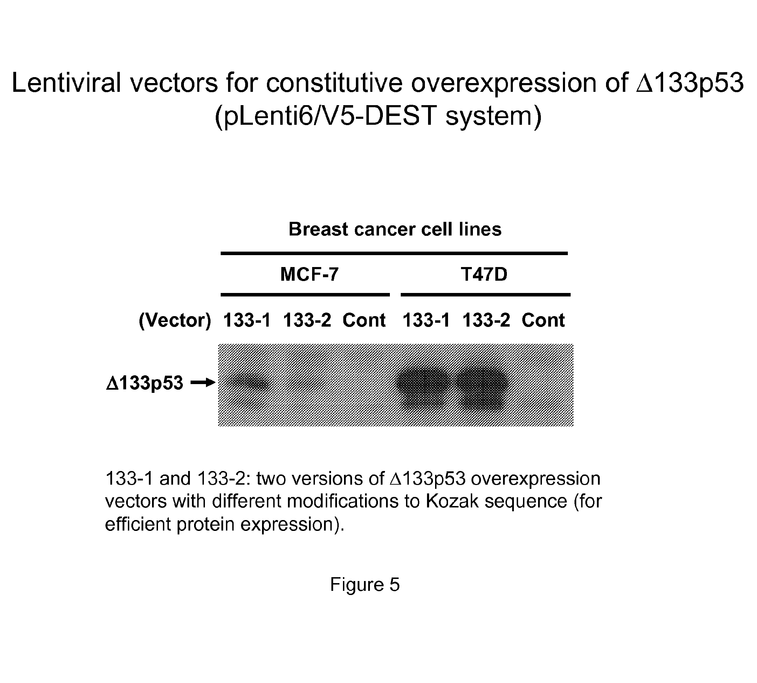

Lentiviral constructs to express Δ133p53 were generated using a pLenti6/VS-DEST expression vectors (Invitrogen). Figure 5 provides data showing that two version of the Δ133p53 vector efficiently expressed the protein.

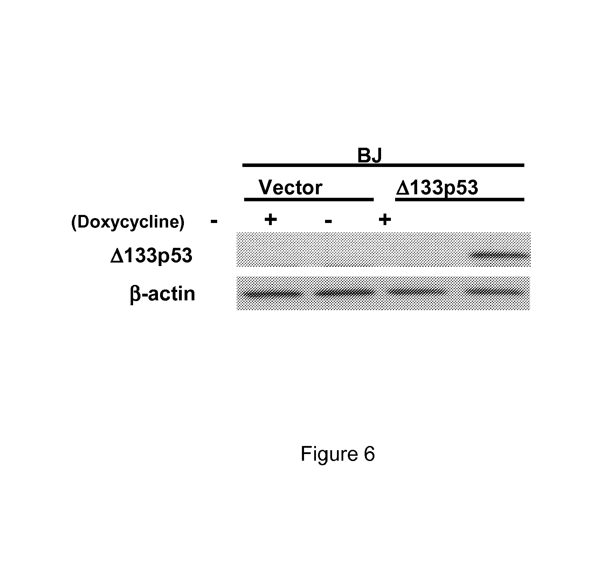

Figure 6 provides data showing establishment of an inducible lentiviral vector system for Δ133p53 expression. Addition of doxycycline induced Δ133p53 expression in BJ fibroblasts.

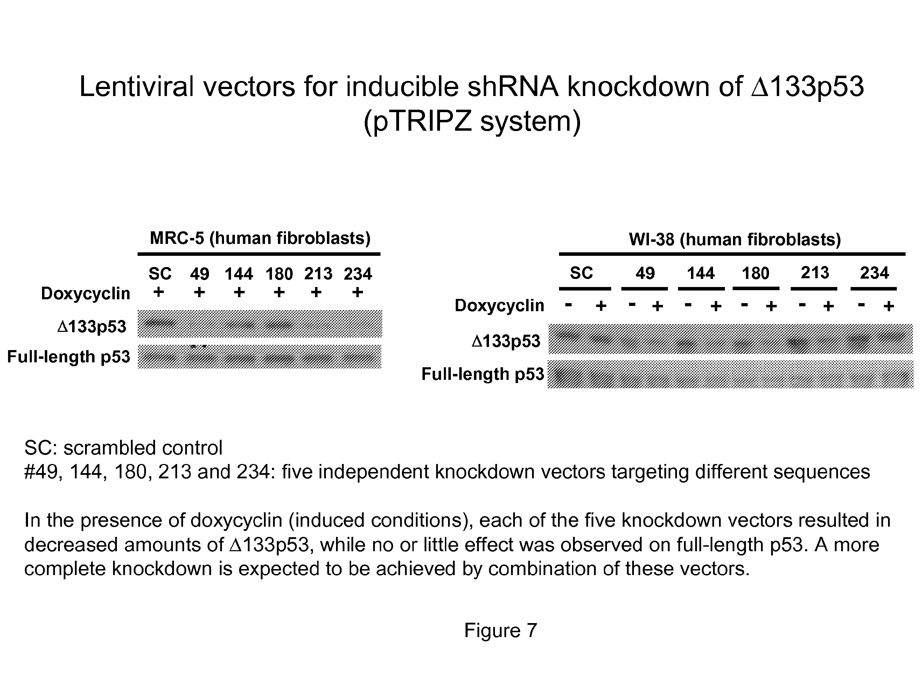

Lentiviral vectors were also generated for inducible shRNA knockdown of Δ133p53 using the pTRIPz system. Figure 7 provides data showing the results of knockdown experiments obtained using five independent knockdown vectors targeting different sequences. In the presence of doxycyclin (induced conditions), each of the five knockdown vectors resulted in decreased amounts of dΔ133p53, while no or little effects was observed on full-length p53.

The singular terms "a," "an," and "the" include plural referents unless context clearly indicates otherwise. The term "plurality" refers to two or more. It is further to be understood that all base sizes or amino acid sizes, and all molecular weight or molecular mass values, given for nucleic acids or polypeptides are approximate, and are provided for description.

The above examples are provided to illustrate the invention but not to limit its scope. Other variants of the invention will be readily apparent to one of ordinary skill in the art and are encompassed by the appended claims.

## Claims

1. A method of increasing the number of induced pluripotent stem cells (iPS) obtained from somatic cells that express at least one reprogramming factor, the method comprising increasing the Δ133p53 levels in the somatic cells, thereby increasing the number of iPS obtained from the somatic cells.

2. The method of claim 1, wherein the level of expression of Δ133p53 is increased by expressing a recombinant nucleic acid sequence that encodes Δ133p53 in the somatic cells.

3. The method of claim 2, wherein:
(a) the recombinant nucleic acid sequence that encodes Δ133p53 is operably linked to an inducible promoter;
(b) the recombinant nucleic acid sequence is comprised by a plasmid vector, an adenoviral vector or a retroviral vector, optionally a lentiviral vector ; or
(c) the recombinant nucleic acid is present in an expression cassette that comprises lox recombination sites.

4. The method of claim 1, 2 or 3, wherein the somatic cells are fibroblasts.

5. The method of claim 1, wherein the level of expression of Δ133p53 is increased by introducing exogenous Δ133p53 into the cell.

6. The method of any one of the preceding claims, wherein:
(a) the reprogramming factor is selected from the group consisting of OCT4, KLF4, SOX2, and c-myc;
(b) the somatic cells express OCT4, KLF4, and SOX2; or
(c) the somatic cells express OCT4, KLF4, SOX2, and c-myc.

7. An isolated iPS cell comprising an expression vector encoding a recombinant Δ133p53, wherein the iPS cell expresses at least one re-programming growth factor.

8. The isolated iPS cell of claim 7, wherein the recombinant nucleic acid sequence that encodes Δ133p53 is operably linked to an inducible promoter.

9. The isolated iPS cell of claim 7 or 8, wherein the expression vector is a plasmid vector, an adenoviral vector or a retroviral vector, optionally a lentiviral vector .

10. The isolated iPS cell of claim 7, 8 or 9, wherein:
(a) the reprogramming factor is selected from the group consisting of OCT4, KLF4, SOX2, and c-myc;
(b) wherein the somatic cells express OCT4, KLF4, and SOX2; or
(c) the somatic cells express OCT4, KLF4, SOX2, and c-myc.

11. The isolated iPS cell of claim 7, 8, 9 or 10, wherein the expression vector comprises a lox recombination site.

12. A method of differentiating an iPS cell of claim 11, the method comprising suppressing expression of Δ133p53.

13. The method of claim 12, wherein the method comprises expressing a cre recombinase to disrupt expression of Δ133p53, wherein the nucleic acid encoding Δ133p53 comprises a lox recombination site.

## Patentansprüche

1. Verfahren zur Erhöhung der Anzahl von induzierten pluripotenten Stammzellen (iPS), erhalten aus somatischen Zellen, welche wenigstens einen Umprogrammierungsfaktor exprimieren, wobei das Verfahren die Erhöhung der Δ133p53-Level in den somatischen Zellen umfasst, wodurch sich die Anzahl von aus den somatischen Zellen erhaltenen iPS erhöht.

2. Verfahren gemäß Anspruch 1, wobei der Expressionslevel von Δ133p53 durch die Expression einer rekombinanten Nucleinsäuresequenz, welche Δ133p53 in den somatischen Zellen kodiert, erhöht wird.

3. Verfahren gemäß Anspruch 2, wobei
(a) die rekombinante Nucleinsäuresequenz, welche Δ133p53 kodiert, funktional verknüpft ist mit einem induzierbaren Promotor;
(b) die rekombinante Nucleinsäuresequenz in einem Plasmidvektor, einem adenoviralen Vektor oder einem retroviralen Vektor, gegebenenfalls einem lentiviralen Vektor enthalten ist; oder
(c) die rekombinante Nucleinsäure in einer Expressionskassette, welche lox-Rekombinationsstellen umfasst, anwesend ist.

4. Verfahren gemäß Anspruch 1, 2 oder 3, wobei die somatischen Zellen Fibroblasten sind.

5. Verfahren gemäß Anspruch 1, wobei der Expressionslevel von Δ133p53 durch die Einführung von exogenem Δ133p53 in die Zelle erhöht wird.

6. Verfahren gemäß einem der vorherigen Ansprüche, wobei
(a) der Umprogrammierungsfaktor ausgewählt ist aus der Gruppe, bestehend aus OCT4, KLF4, SOX2 und c-myc;
(b) die somatischen Zellen OCT4, KLF4 und SOX2 exprimieren; oder
(c) die somatischen Zellen OCT4, KLF4, SOX2 und c-myc exprimieren.

7. Isolierte iPS-Zelle, umfassend einen Expressionsvektor, welcher ein rekombinantes Δ133p53 kodiert, wobei die iPS-Zelle wenigstens einen umprogrammierenden Wachstumsfaktor exprimiert.

8. Isolierte iPS-Zelle gemäß Anspruch 7, wobei die rekombinante Nucleinsäuresequenz, welche Δ133p53 kodiert, funktional verknüpft ist mit einem induzierbaren Promotor.

9. Isolierte iPS-Zelle gemäß Anspruch 7 oder 8, wobei der Expressionsvektor ein Plasmidvektor, ein adenoviraler Vektor oder ein retroviraler Vektor, gegebenenfalls ein lentiviraler Vektor ist.

10. Isolierte iPS-Zelle gemäß Anspruch 7, 8 oder 9, wobei
(a) der Umprogrammierungsfaktor ausgewählt ist aus der Gruppe, bestehend aus OCT4, KLF4, SOX2 und c-myc;
(b) wobei die somatischen Zellen OCT4, KLF4 und SOX2 exprimieren; oder
(c) die somatischen Zellen OCT4, KLF4, SOX2 und c-myc exprimieren.

11. Isolierte iPS-Zelle gemäß Anspruch 7, 8, 9 oder 10, wobei der Expressionsvektor eine lox-Rekombinationsstelle umfasst.

12. Verfahren zur Differenzierung einer iPS-Zelle gemäß Anspruch 11, wobei das Verfahren die Suppression der Expression von Δ133p53 umfasst.

13. Verfahren gemäß Anspruch 12, wobei das Verfahren die Expression einer Cre-Rekombinase umfasst, um die Expression von Δ133p53 zu unterbinden, wobei die Nucleinsäure, welche Δ133p53 kodiert, eine lox-Rekombinationsstelle umfasst.

## Revendications

1. Procédé d'augmentation du nombre de cellules souches pluripotentes induites (CSPI) obtenues à partir de cellules somatiques qui expriment au moins un facteur de reprogrammation, lequel procédé comporte le fait d'augmenter les quantités de Δ133p53 dans les cellules somatiques, ce qui permet d'augmenter le nombre de CSPI obtenues à partir de ces cellules somatiques.

2. Procédé selon la revendication 1, dans lequel on augmente le degré d'expression de Δ133p53 par expression d'une séquence d'acide nucléique recombinant qui code pour Δ133p53 dans les cellules somatiques.

3. Procédé selon la revendication 2, dans lequel :
(a) la séquence d'acide nucléique recombinant qui code pour Δ133p53 est liée de manière fonctionnelle à un promoteur inductible ;
(b) la séquence d'acide nucléique recombinant comporte un vecteur plasmidique, un vecteur adénoviral ou un vecteur rétroviral, en option un vecteur lentiviral ;
(c) ou l'acide nucléique recombinant se trouve dans une cassette d'expression qui comprend des sites de recombinaison lox.

4. Procédé selon la revendication 1, 2 ou 3, dans lequel les cellules somatiques sont des fibroblastes.

5. Procédé selon la revendication 1, dans lequel on augmente le degré d'expression de Δ133p53 par introduction de Δ133p53 exogène à l'intérieur de la cellule.

6. Procédé selon l'une quelconque des revendications précédentes, dans lequel :
(a) le facteur de reprogrammation est choisi dans l'ensemble constitué par OCT4, KLF4, SOX2 et c-myc ;
(b) les cellules somatiques expriment OCT4, KLF4 et SOX2 ;
(c) ou les cellules somatiques expriment OCT4, KLF4, SOX2 et c-myc.

7. Cellule souche pluripotente induite isolée comprenant un vecteur d'expression qui code pour une protéine Δ133p53 recombinante, laquelle cellule souche pluripotente induite exprime au moins un facteur de croissance et de reprogrammation.

8. Cellule souche pluripotente induite isolée selon la revendication 7, dans laquelle la séquence d'acide nucléique recombinant qui code pour Δ133p53 est liée de manière fonctionnelle à un promoteur inductible.

9. Cellule souche pluripotente induite isolée selon la revendication 7 ou 8, dans laquelle le vecteur d'expression est un vecteur plasmidique, un vecteur adénoviral ou un vecteur rétroviral, en option un vecteur lentiviral.

10. Cellule souche pluripotente induite isolée selon la revendication 7, 8 ou 9, dans laquelle :
(a) le facteur de reprogrammation est choisi dans l'ensemble constitué par OCT4, KLF4, SOX2 et c-myc ;
et dans laquelle
(b) les cellules somatiques expriment OCT4, KLF4 et SOX2 ;
(c) ou les cellules somatiques expriment OCT4, KLF4, SOX2 et c-myc.

11. Cellule souche pluripotente induite isolée selon la revendication 7, 8, 9 ou 10, dans laquelle le vecteur d'expression comprend un site de recombinaison lox.

12. Procédé de différenciation d'une cellule souche pluripotente induite selon la revendication 11, qui comprend le fait de supprimer l'expression de Δ133p53.

13. Procédé selon la revendication 12, qui comprend le fait d'exprimer une recombinase cre pour interrompre l'expression de Δ133p53, étant entendu que l'acide nucléique qui code pour Δ133p53 comprend un site de recombinaison lox.
